(19) Europäisches Patentamt
European Patent Office
Office européen des brevets

(11) Numéro de publication: **0 090 901**
**B1**

(12) # FASCICULE DE BREVET EUROPEEN

(45) Date de publication du fascicule du brevet: **15.07.87**

(51) Int. Cl.⁴: **G 01 N 33/14**, G 01 N 24/08, G 01 N 24/04

(21) Numéro de dépôt: **82402209.9**

(22) Date de dépôt: **03.12.82**

(54) **Détection et différenciation des molécules naturellement deutériées et application, notamment, à la détection de la chaptalisation des vins.**

(30) Priorité: **04.12.81 FR 8122710**

(43) Date de publication de la demande: **12.10.83 Bulletin 83/41**

(45) Mention de la délivrance du brevet: **15.07.87 Bulletin 87/29**

(84) Etats contractants désignés: **DE FR GB IT**

(56) Documents cités:
**FR - A - 2 475 736**
**US - A - 4 206 132**

**SOV. PHYS.-JETP, vol. 42, no. 6, décembre 1975, pages 950-954, New York, USA, YU.I. NERONOV et al.: "NMR study of hydrogen isotopes to determine the ratio of the proton and deuteron magnetic moments to the eighth decimal place"**
**Z. NATURFORSCH., vol. 34C, 2 novembre 1978, pages 1-4, Wiesbaden, DE., P. RAUSCHENBACH et al.: "Vergleich der Deuterium- und Kohlenstoff-13-Gehalte in Fermentations- und Syntheseethanol"**

(73) Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Martin, Gérard Jean, 21, Rue Thomas Maisonneuve, F-44000 Nantes (FR)**
Inventeur: **Martin née Martin, Maryvonne Lucie, 21, Rue Thomas Maisonneuve, F-44000 Nantes (FR)**

(74) Mandataire: **Gillard, Marie-Louise et al, Cabinet Beau de Loménie 55, Rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 090 901 B1

ACTORUM AG

**Description**

La présente invention est relative à un procédé de détection et de différentiation qualitative et quantitative des molécules naturellement deutériées, ainsi qu'à un dispositif pour la mise en œuvre de ce procédé.

Le deutérium D (l'isotope naturel de l'hydrogène) est universellement répandu puisqu'il accompagne pratiquement toujours l'hydrogène.

Si sur la terre, sa répartition moyenne est très faible (de l'ordre de 0,015%, c'est-à-dire 150 ppm), elle subit toutefois des fluctuations importantes par suite des différents facteurs de fractionnement isotopique d'origine géologique, géographique, climatologique, physique, chimique et biochimique, qui interviennent dans l'histoire d'une molécule.

L'étalon international, défini à VIENNE, est un échantillon d'eau des océans (Standard Mean Ocean Water ou SMOW) qui contient 155,78 ppm de D/H. Par contre, de l'eau extraite de la glace antarctique (Standard Light Antarctique Precipitation) ne contient que 89,0 ppm de deutérium, soit −42,8%. C'est ainsi que la plupart des molécules contiennent des quantités variables de deutérium. Par exemple, les sucres sont en général plus riches que le SMOW (D/H 155 à 175 ppm), mais leurs produits de fermentation notamment l'éthanol, sont plus pauvres (D/H = 135 à 115 ppm). Les lipides sont également appauvris en deutérium par rapport au SMOW, et le méthanol ne contient pas plus de 90 ppm de deutérium.

L'origine de ces variations, ainsi que les mécanismes qui les gouvernent ne sont pas encore bien connus. Ce n'est pas surprenant si on considère que la seule méthode qui permettait jusqu'à maintenant de mesurer les teneurs globales en deutérium, la spectrographie de masse, est une technique destructive qui nécessite la combustion de l'échantillon et la transformation en eau de tous les atomes d'hydrogène.

Pouvoir donc mesurer avec précision la quantité de D présent dans une molécule, c'est pouvoir déterminer son origine géographique, chimique, biologique, climatologique, etc... Comme, d'autre part la plupart des molécules ont une pluralité d'atomes d'hydrogène (et de son isotope naturel le deutérium qui est constitué d'un proton et d'un neutron), pouvoir déterminer les taux intramoléculaires de deutérium, c'est pouvoir dresser une véritable carte d'identité d'une molécule, et reconnaître avec une très grande précision sa provenance.

Les applications de ce procédé peuvent être innombrables: outre la détection de la chaptalisation des vins basée sur la reconnaissance de l'origine de l'éthanol présent dans le vin, on peut encore citer:

– la reconnaissance de l'origine des vodkas, whiskies, gins, eaux de vie de céréales et plantes fourragères,

– la reconnaissance de l'origine des rhums, marcs, cognacs, eaux de vie de fruits,

– le contrôle de la provenance géographique des anétholes et estragoles,

– le contrôle de la provenance géographique du pétrole,

– la différenciation des caoutchoucs (entre le caoutchouc synthétique et le caoutchouc naturel),

– la détermination de l'origine géographique de l'eau, etc...

En fait, la détermination de l'origine biologique, chimique ou géographique des molécules (par exemple de l'alcool éthylique) constituait jusqu'à maintenant un problème non résolu, ou très mal résolu. Ainsi:

– H. SIMON et Collab. [Lebensmittel Forsch. p. 136 (1975)] préconisaient un marquage radioactif au carbone 14, lequel marquage permettait d'effectuer une identification dans certains cas.

– J. BRICOUT [Rev. Cytol. Biol. Veget. Bot. 1, 133 (1978)] et

– A. RAUSCHENBACH et Collab. [Z. Naturforsch. 34c, p. 1 (1979)] ont démontré qu'il est aussi possible dans certaines conditions de mesurer par spectrométrie de masse la teneur statistique moyenne de toute la molécule d'éthanol, en deutérium ($^2H$), oxygène ($^{18}O$) et carbone ($^{13}C$), ce qui permet la distinction entre l'éthanol de synthèse et l'éthanol naturel.

En ce qui concerne la chaptalisation, le procédé dit «de l'extrait sec» (Décret du 19 Avril 1898 et Article 8 du Code du Vin) a été utilisé pour tenter de détecter les vins suralcoolisés. Lorsque le rapport de la teneur en alcool à celle de l'extrait réduit est supérieur à 4,6 (vins rouges) ou 6,5 (vins blancs), les vins correspondants sont présumés être suralcoolisés. Ce procédé a été repris sous une forme un peu différente dans un règlement de la Communauté Economique Européenne n° 2984 de 1978 (JOCE L 360 du 22 Décembre 1978, p. 31). Il s'agit dans ce cas du rapport densimétrique alcool/extrait sec. Cependant, il est possible d'affirmer aujourd'hui, après plusieurs années de pratique, que tous ces procédés sont peu fiables et assez peu reproductibles. D'autre part, il est actuellement impossible de distinguer l'origine naturelle d'éthanols provenant de la fermentation de différents végétaux contenant des polysaccharides (céréales diverses, betteraves, pommes de terre, fruits variés, etc...). Par voie de conséquence, l'estimation quantitative de mélanges de ces alcools est impossible. Il en est de même pour le dosage des alcools de synthèse par rapport aux alcools naturels. Si même on arrive à faire la distinction par spectrométrie de masse, le dosage est très imprécis. De plus, la procédure utilisée est longue et fastidieuse.

La faible abondance naturelle du deutérium explique le fait que ce noyau est $10^6$ fois plus difficile à détecter que le proton, pour un volume égal d'échantillon. C'est pourquoi l'usage de spectromètres à haut champ et large diamètre de la bobine supraconductrice, est nécessaire. Ce n'est cependant pas une condition suffisante pour effectuer des mesures quantitatives significatives et reproductibles, but que s'est précisément assignée la présente invention.

La présente invention a pour objet un procédé de détection et de différenciation qualitative et quantitative des molécules naturellement deutériées, caractérisè en ce que:

a) l'on prépare un étalon en mélangeant un produit hydrocarboné du commerce avec son analogue deutérié artificiellement à 95–99%, de manière à ce que le taux de deutériation global du mélange soit compris entre 0,01 et 0,2%,

b) l'on introduit cet étalon dans une cellule de mesure de l'appareil d'analyse par résonance magnétique nucléaire (RMN), ladite cellule étant constituée de telle façon qu'elle puisse recevoir l'étalon de manière à ce qu'il n'y ait aucun mélange entre l'étalon et le produit à analyser,

c) l'on ajoute dans la cellule le produit à analyser, et

d) l'on introduit la cellule dans l'appareil RMN (D) et on enregistre le spectre du deutérium que l'on compare au spectre RMN (D) des produits de provenance géographique et de sources chimiques et/ou biochimiques connues, réalisé auparavant avec le même étalon.

Il faut noter que:

– l'étalon peut être constitué par une référence intramoléculaire dans le cas des alcools. Cette référence est chimiquement combinèe à l'alcool et le produit résultant est introduit dans la cellule selon b) ci-dessus;

– pour déterminer la pureté énantiomérique des alcools, on combine chimiquement l'alcool à un composé optiquement actif et on introduit ce nouveau composé dans la cellule selon b) cidessus.

Selon un mode de réalisation particulièrement avantageux de l'objet de l'invention, la référence intramoléculaire est constituée à partir d'anhydride acètique et/ou d'acide acétique et/ou de chlorure d'acétyle et/ou de bromure d'acétyle, le produit résultant est alors de l'acétate d'éthyle.

Selon un mode de réalisation particulièrement avantageux de l'objet de l'invention, le composé optiquement actif est constitué d'acide camphanique et/ou d'acide malique et/ou d'acide aspartique et/ou de proline et/ou de phénylalanine, auxquels on ajoute des quantités faibles de chelates.

Selon un mode de réalisation avantageux du procédé objet de la présente invention, on choisit l'étalon parmi les produits du groupe qui comprend le tétraméthylsilane, l'hexaméthyldisilane, l'hexaméthyldisiloxane, l'hexaméthyldisilazane, le diméthyl-4,4-silapentane-4-sulfonate de sodium, le diméthyl-4,4-silapentane-4-carboxylate de sodium, l'octaméthylcyclotétrasiloxane, le tétrakis-(triméthylsilyl)méthane, l'acétonitrile, l'acétone, le benzène, le diméthylsulfoxyde et le chloroforme.

Selon un autre mode de réalisation avantageux du procédé objet de la présente invention, on ajoute à l'étalon et/ou à l'échantillon à analyser, des quantités faibles de chélates du chrome et/ou du fer et/ou du cobalt et/ou du nickel et/ou d'europium et/ou du praséodyme et/ou d'ytterbium et/ou de dysprosium et/ou du holmium et/ou d'erbium.

Suivant une modalité particulière de ce mode de réalisation, les chélates précédents sont préparés à partir d'une dione de formule générale I ci-après:

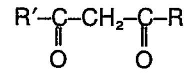

(I)

dans laquelle:

R représente: $CH_3$, $tC_4H_9$, $nC_3F_7$, $C_2F_5$, $CF_3$, $tC_4D_9$, et

R′ représente: $CH_3$, $tC_4H_9$, $CF_3$, $nC_3F_7$, $C_2F_5$, $nC_3H_7$.

La précision des mesures RMN peut être accrue si on ajoute un catalyseur qui favorise l'échange des sites hydroxyles de l'eau et de l'alcool. En effet, le phénomène de l'échange chimique des protons et deutérons hydroxyliques doit être considèré avec soin: la moindre trace d'ions $H_3O^+$ catalyse cet échange, et il en résulte des variations de largeur de raie des sites OD. Cet échange chimique peut affecter indirectement la largeur de la raie CHD, car la relaxation du deutérium méthylénique est susceptible de dépendre aussi de la vitesse d'échange (relaxation par couplage scalaire), ce qui constitue une source d'imprécision. La vitesse d'échange dépend elle-même de la pureté de l'alcool, conditionnée par la méthode d'échantillonage utilisée.

Conformément à l'invention, et dans le cas où l'analyse porte sur des vins pour détecter les taux des molécules d'éthanol naturellement deutériées, on procède auparavant à une distillation après la neutralisation à pH 7,5 à l'aide d'un alcali.

Selon un autre mode de réalisation du procédé objet de la présente invention, on déshydrate l'échantillon sur du $CaH_2$ et on distille par cryopiégeage à l'azote liquide les alcools humides.

Selon un autre mode de réalisation du procédé objet de la présente invention, on ajoute à l'échantillon entre 0,01 et 1% vol/vol d'un acide pris dans le groupe qui comprend l'acide chlorhydrique, l'acide nitrique, l'acide sulfurique, l'acide phosphorique, l'acide trichloracétique et l'acide perchlorique.

La présente invention est également relative à une cellule de mesure par RMN, caractérisée en ce qu'elle est constituée de deux tubes coaxiaux isolés l'un de l'autre, et dont le rapport

$$\frac{\text{diamètre tube extérieur}}{\text{diamètre tube intérieur}}$$

est compris entre 2,5:1 à 3,5:1. Le tube intérieur maintenu solidaire du tube extérieur par des joints de solidarité est destiné à recevoir l'étalon, et peut être scellé après l'introduction de celui-ci, tandis que le tube extérieur est destiné à recevoir la substance à analyser. Celle-ci est introduite dans la partie inférieure dudit tube extérieur à travers un orifice percé dans le joint de solidarité qui maintient les deux tubes.

Selon un mode de réalisation particulièrement avantageux de la cellule objet de la présente invention, celle-ci est dotée d'un guide gradué et

étalonné en fonction du spectromètre utilisé, lequel guide permet de régler la hauteur de la turbine sur le tube extérieur selon la quantité de la substance contenue dans la partie inférieure de ce tube extérieur.

Selon un autre mode de réalisation avantageux de l'objet de l'invention, les deux tubes coaxiaux sont en verre pyrex calibré.

Conformément à l'invention, les joints de solidarité sont au nombre de deux, l'un inférieur maintenant le tube intérieur sur sa base, et l'autre supérieur maintenant le tube intérieur en son milieu.

Selon un mode de réalisation avantageux de l'objet de l'invention, les deux joints de solidarité sont constitués de deux blocs de Téflon cylindriques.

Selon un autre mode de réalisation avantageux de l'objet de l'invention, les deux blocs sont percés: le bloc supérieur de deux orifices, l'un pour l'introduction de la substance à analyser et l'autre pour l'échappement de l'air, tandis que le bloc inférieur est percé d'un orifice pour fixer le tube intérieur contenant la substance étalon. Des saignées cylindriques sont en outre usinées sur lesdits blocs, afin de permettre un glissement aisé dans le tube extérieur.

Selon un mode de réalisation particulièrement avantageux de l'objet de l'invention, deux tiges filetées creuses et solidaires l'une de l'autre, sont adaptables sur le bloc supérieur de solidarité, de manière à régler exactement l'affleurement de la substance à analyser contenue dans la partie inférieure du tube extérieur, à la surface inférieure dudit bloc de solidarité.

Conformément à l'invention, le diamètre des tubes coaxiaux est compris entre 10 et 50 mm pour le tube extérieur et entre 4 et 12 mm pour le tube intérieur.

Outre les dispositions qui précèdent, l'invention comprend encore d'autres dispositions qui ressortiront de la description qui va suivre.

L'invention pourra être mieux comprise à l'aide du complément de description suivant, qui se réfère à des exemples de mise en œuvre du procédé objet de la présente invention à l'aide du dispositif qui fait également l'objet de la présente invention, lequel dispositif est représenté schématiquement, à titre d'exemple non limitatif, dans les dessins annexés.

Il doit être bien entendu, toutefois, que les exemples décrits ci-après, la représentation des paramètres utilisés, ainsi que le dispositif décrit dans ce qui va suivre et représenté aux dessins, sont donnés uniquement à titre d'illustration de l'objet de l'invention, mais n'en constituent en aucune manière une limitation.

1. Représentation des paramètres utilisés

Le spectre RMN du deutérium d'un échantillon donné, est constitué de plusieurs signaux (i); la substance étalon donne un seul signal (o). Il convient donc d'exprimer l'intensité d'un signal au moyen d'un paramètre précis. On a utilisé dans ce qui va suivre, les hauteurs des signaux h(i), quand les largeurs à mi-hauteur étaient égales (ou constantes), car les hauteurs sont mesurables avec précision. Dans les autres cas, on a utilisé les surfaces intégrées S(i). Ces deux paramètres constituent deux échelles différentes, mais très corrélées entre elles. Cependant, la hauteur h(i) ou la surface S(i) d'un signal i peuvent varier légèrement d'un spectre à l'autre, et il est nécessaire de considérer alors les rapports D

$$D(i) = \frac{h(i)}{h(o)} \text{ et } D'(i) = \frac{S(i)}{S(o)}$$

lesquels, eux, ne dépendent plus des conditions expérimentales. h(o) et S(o) représentent le signal étalon.

Il en est de même pour

$$T = \sum_{i=1}^{n} D(i)$$

où n représente le nombre de molécules différentes dans le mélange, et

T représente le taux global.

2. Etude d'un éthanol

Les taux intramoléculaires relatifs de deutérium sont définis de la façon suivante. Le signal de référence est celui du groupe méthyle auquel on attribue un poids statistique de 3. On compare ensuite l'intensité du groupe méthylène h (CHD) à celle de l'intensité normalisée du groupe méthyle, soit $\frac{h(CH_2D)}{3}$ ; le taux R intramoléculaire relatif s'exprime alors par:

$$R_h(CHD) = \frac{3\ h(CHD)}{h(CH_2D)} \text{ et } R_S(CHD) = \frac{3\ S(CHD)}{S(CH_2D)}$$

Les indices h ou S indiquent que les hauteurs ou les surfaces intégrées ont été respectivement utilisées pour représenter les quantités de noyaux résonant à une position donnée.

Dans le cas du site hydroxyle, la surface $S_{OH}$ seule peut être valablement considérée, car par suite des trop grandes fluctuations des largeurs de raies, les intensités ne sont plus comparables directement.

On remarquera que si le deutérium était réparti statistiquement dans la molécule, les paramètres définis seraient:

$R_h(CHD) = R_S(SHD) = 2$, ce qui correspond à la distribution méthylène/méthyle = 2/3. Une valeur 2,5 par exemple indique donc une surpopulation relative du méthylène (ou une sous-population du méthyle) considérable.

Il est à noter également que si hauteurs et surfaces des signaux étaient toutes deux exactement proportionnelles au nombre de noyaux résonant à la fréquence considérée, les rapports $R_h$ et $R_S$ devraient être égaux. Cependant, cette égalité exige que les largeurs à mi-hauteur des raies méthyle et méthylène soient rigoureusement égales, ce qui peut ne pas être le cas (les largeurs de raies sont gouvernées par des processus de relaxation susceptibles de s'effectuer à des vitesses légèrement différentes dans les deux sites). Il en résulte que les valeurs de $R_h$ sont souvent assez nettement supérieures aux valeurs de $R_S$.

L'introduction d'une substance étalon dans le tube coaxial intérieur, permet une comparaison des taux globaux de deutériation. Cet emploi d'une référence permet de définir deux autres paramètres complémentaires $R_h$ (Ref) et $R_S$ (Ref).

$$R_h(Ref) = \frac{3\ h(Ref)}{h(CH_2D)} \qquad R_S(Ref) = \frac{3\ S(Ref)}{S(CH_2D)}$$

ainsi qu'un taux global relatif:

$$T_s = \frac{S(CH_2D) + S(CHD) + S(OD)}{S(Ref)}$$

Grâce à ces valeurs, on peut aussi comparer la variation du taux de deutérium partiel (R) ou global (T) d'un échantillon à un autre.

Une autre façon de mesurer le taux de deutérium global d'une molécule d'éthanol sans être gêné par la contribution des molécules deutériées contenant des sites échangeables avec l'eau ($CH_3CH_2OD$), est d'utiliser une référence intramoléculaire. Les échantillons d'alcool à comparer sont transformés en acétate d'éthyle par réaction avec un excès d'anhydride acétique référence (Ref), d'origine contrôlée et de teneur en deutérium (D/H) connue. On peut alors doser aisément les teneurs $(D/H)_I$ et $(D/H)_{II}$ des sites $CH_2DCH_2OH$ et $CH_3CHDOH$ par rapport à celle de l'anhydride acétique en utilisant les paramètres $R_h$ qui sont accessibles avec une grande précision. On aura alors:

$$R_h(CH_2D/Ref) = \frac{3h(CH_2D)}{h(Ref)}$$

$$R_h(CHD/Ref) = \frac{3h(CHD)}{h(Ref)}$$

3. Représentation physique des taux intramoléculaires relatifs

Le spectre protonique de l'éthanol est caractérisé par le système triplet-quartet-singulet (dans le cas de l'échange rapide des protons hydroxyliques qui intervient généralement lorsque les alcools sont imparfaitement purifiés). Ce système correspond à la molécule réelle $CH_3CH_2OH$.

La situation est très différente quand on considère les molécules deutériées naturellement, car, par suite de la très faible abondance naturelle de $^2H$ (0,015%), il y a une probabilité très faible ($\simeq 2.10^{-8}$) pour que deux atomes de deutérium soient présents dans la même molécule. La RMN $^2H$ observera donc les signaux des molécules suivantes:

$$\underset{(1)}{CH_2DCH_2OH}\ ,\quad \underset{(2)}{CH_3CHDOH}\ ,\quad \underset{(3)}{CH_3CH_2OD}$$

Chacune de ces trois molécules est caractérisée par:

(1) : $h_{(1)}\ S_{(1)}$

(2) : $h_{(2)}\ S_{(2)}$

(3) : $h_{(3)}\ S_{(3)}$

Ainsi: $D_{(1)} = \frac{h(1)}{h(0)}$ , $D_{(2)} = \frac{h(2)}{h(0)}$ , $D_{(3)} = \frac{h(3)}{h(0)}$

et le taux intramoléculaire relatif

$$R = \frac{3\ D(2)}{D(1)}$$

De même, le taux global est égal:

$$T = \frac{S(1) + S(2) + S(3)}{S(o)}$$

Les taux de deutérium définis pourraient aisément être convertis en fractions molaires des différentes molécules (1), (2), (3). En effet, en appelant fm(i) la fraction molaire de l'espèce i, nous aurons:

$$fm(1) = \frac{S\ (CH_2D)}{\Sigma S} \quad fm(2) = \frac{S(CHD)}{\Sigma S} \quad fm(3) = \frac{S(OD)}{\Sigma S}$$

avec $\Sigma S = S(CH_2D) + S(CHD) + S(OD)$ (les S désignent toujours les surfaces des signaux spécifiés).

Les expressions analogues peuvent être écrites pour les intensités i, mais elles ne sont pas utilisables dans ce cas, car la raie OD est beaucoup plus large que les autres. On voit donc que

$$R(CHD) = \frac{3fm(2)}{fm(1)}$$

Il est possible de connaître la quantité de deutérium présente dans chacune des molécules (1), (2) et (3) en multipliant les fractions molaires fm(i) par le taux global $T_S$.

4. Relation entre la distribution interne de deutérium et le taux de chaptalisation d'un vin

a) Rappel du symbolisme utilisé

Molécules réelles:

$$\underset{(1)}{CH_2DCH_2OH},\ \underset{(2)}{CH_3CHDOH},\ \underset{(3)}{CH_3CH_2OD}$$

Taux intramoléculaire de deutérium: $R = 3\ \frac{A(2)}{A(1)}$

A peut être soit la hauteur h, soit la surface S

Origine des éthanols:

| | |
|---|---|
| vin | : V |
| sucre de betterave | : B |
| mélange vin + sucre de betterave | : M |

b) Détermination de la relation entre $R^M$ et $R^V$, $R^B$

On considère les molécules (1) et (2) seulement. Un mélange d'alcool de vin et d'alcool de betterave sera défini par la fraction molaire $F_D{}^B$, c'est-à-dire:

$$F_D{}^B = (n_1{}^F + n_2{}^B) / (n_1{}^B + n_2{}^F) + (n_1{}^V + n_2{}^V)$$

$n_1^B$ nombre de moles I venant de la betterave, etc...

D'autre part, le taux intramoléculaire de deutérium du mélange $R^M$ est égal à:

$$\frac{3A^M_{(2)}}{A^M_{(1)}}$$

c'est-à-dire

$$R^M = 3\,\frac{(n_2^V + n_2^B)}{(n_1^V + n_1^B)}$$

Par arrangement de cette équation, on trouve

$$R^M = \frac{R^V}{1 + n_1^B/n_1^V} + \frac{R^B}{1 + n_1^V/n_1^B}$$

c'est-à-dire, compte tenu de la définition de la fraction molaire F et de $a_D = Ta_H$

$$R^M = \frac{R^B + a_H TKR^V}{(1 + a_H TK)}$$

où T est la teneur relative en deutérium de l'alcool de betterave par rapport à l'alcool de vin.

$$K = \frac{3 + R^V}{3 + R^B} \quad a_D = \frac{F_D^V}{F_D^B} \text{ et } a_H = \frac{F_H^V}{F_H^B}$$

(rapport exprimé en volume)

La variation de la fonction $R^M$ avec $a_H$, pour $R^V$ et $R^B$ fixés, n'est pas linéaire, mais ne s'écarte pas très sensiblement ($\pm$ 1°/°° de la linéarité.

En première approximation on pourra considérer que:

$$R^M = (1 - F_H{}^B)\,R^V + F_H{}^B\,R^B$$

c) Application au taux de chaptalisation d'un vin

Du point de vue de la RMN, un vin chapitalisé peut être considéré comme un mélange d'éthanols deutériés (1) et (2) (et (3)) dont une partie provient du raisin et l'autre de la betterave. (Dans l'hypothèse d'une chaptalisation au sucre de betterave).

Si on appelle t le titre du vin (en % en volume) c le taux de chaptalisation (en % en volume), on peut écrire simplement:

$$F_H{}^B = c/t$$

Ainsi, le taux de chaptalisation c peut être calculé en fonction de t (aisément mesurable) et de $R^M$, $R^V$ et $R^B$.

$$c = \frac{tT\,(3 + R^B)\,(R^V - R^M)}{(3 + R^V)\,(R^M - R^B) + T(3 + R^B)\,(R^V - R^M)}$$

ou, en première approximation

$$c = \frac{t(R^V - R^M)}{(R^V - R^B)}$$

Une autre représentation de c fait apparaître le paramètre $P_I$

$$c = \frac{t}{P_I\,\frac{(R^B - R^M)}{R^M - R^V} + 1}$$

$$\text{avec} \qquad P_I = \frac{(D/H)_I^B}{(D/H)_I^V}$$

$$\text{et } (D/H)_I = 2\,fm_I\,(D/H)$$

si $P_I$ est voisin de 1, on retrouve l'expression simplifiée précédente.

Il y a lieu de remarquer toutefois que:

1) Dans l'expression précédente, t et $R^M$ sont mesurés sur l'extrait éthanolique du vin chaptalisé, mais $R^V$ et $R^B$ doivent être considérés comme des valeurs d'étalonnage. $R^V$ est le taux de deutérium du vin non chaptalisé et $R^B$ celui de l'alcool de betterave utilisé.

Exemple:

soit un vin à 12° caractérisé par $R^M = 2{,}532$

On a mesuré, sur des vins étalons $R^V = 2{,}485$

$R^B = 2{,}711$

On en déduit c = 2,5°

2) Une autre possibilité d'utiliser la formule précédente, est de comparer un vin chaptalisé inconnu $c_x$ à deux vins étalons, l'un non chaptalisé et l'autre chaptalisé à un taux connu $c_1$

On a

$$C_1 = t_1\left(\frac{R_0^V - R_1^M}{R_0^V - R_0^B}\right)$$

$$c_x = t_x\left(\frac{R_0^V - R_x^M}{R_0^V - R_x^M}\right)$$

$$\text{d'où } c_x = t_x\,\frac{c_1}{t_1}\left(\frac{R_0^V - R_x^M}{R_0^V - R_1^M}\right)$$

$R_0{}^V$ est mesuré sur le vin témoin non chaptalisé, $R_1{}^M$ sur le vin témoin chaptalisé avec un taux $c_1$, et $R_x{}^M$ sur le vin inconnu.

Exemple:

soit un vin non chaptalisé $R_0{}^V = 2{,}460$

un vin chaptalisé à 2,5° et titrant 12°,

$R_1{}^M$ = 2, 532

un vin inconnu titrant 11°, $R_x{}^M = 2{,}507$

On en déduit

$$c_x = \frac{12 \times 2{,}5}{11}\,\frac{(2{,}460 - 2{,}507)}{(2{,}460 - 2{,}532)} = 1{,}5°$$

3) Lorsque la chaptalisation est effectuée au moyen d'un sucre autre que celui de la betterave, les formules précédentes restent valables. Il suffit alors de remplacer $R^B$, le paramètre caractéristique de la betterave par $R^S$ qui est celui du sucre étranger (canne à sucre, glucose de maïs...).

Exemple de calcul dans le cas de la chaptalisation

Chaptalisation d'un vin blanc

Une prise d'essai de 750 ml de vin est neutralisée à pH 7,5 par NaOH 1N et une première distillation est effectuée avec un appareil constitué d'une Colonne Vigreux de 20 cm et d'un réfrigérant de West de 25 cm. On recueille tout ce qui passe avant 90 °C (environ 80 à 150 ml). On rectifie ensuite le distillat avec le même appareil et on recueille 15 ml d'un mélange éthanol-eau qui bout à 78,5° et qui contient 92 à 96% d'éthanol. Ce mélange est introduit dans la cellule de mesure décrite ci-après et contenant de l'acétonitrile comme substance étalon. Le spectre RMN $^2H$ est alors enregistré en utilisant les mêmes paramètres d'acquisition que ceux définis dans l'Exemple précédent.

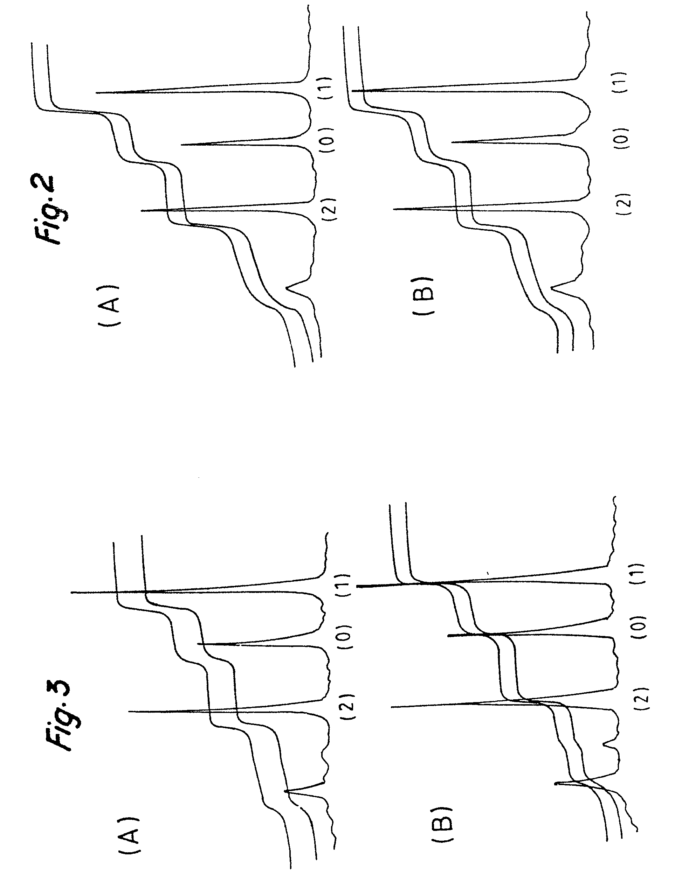

Les figures 3(A) et 3(B) montrent les spectres de deux vins blancs, l'un non chaptalisé (A) et l'autre chaptalisé (B).

Les résultats sont exprimés en utilisant deux échelles différentes, correspondant aux hauteurs (h) et aux surfaces (s) des signaux.

| Signal | | (0) | (1) | (2) |
|---|---|---|---|---|
| A | h | 107,5 | 176,0 | 142,0 |
| | s | 40,5 | 64,8 | 51,0 |
| B | h | 109,0 | 188,0 | 157,0 |
| | s | 34,0 | 51,5 | 42,5 |

On en déduit donc les valeurs suivantes de D(1), D(2) et R

| | $D_1$ | $D_2$ | R |
|---|---|---|---|
| A(h) | 1,637 | 1,321 | 2,421 |
| B(h) | 1,725 | 1,440 | 2,504 |

qui permettent de conclure que B est chaptalisé. (Taux de chaptalisation 1,5°).

Caractérisation des molécules chirales naturellement deutériées: application à la détermination de l'origine des produits.

Les molécules contenant un motif –CHD- peuvent être chirales si les substituants du groupe –CHD- sont différents. Deux cas sont à considérer selon qu'il y a ou non un autre centre de dissymétrie dans la molécule:

a) Cas où il n'y a pas d'autre centre de dissymétrie dans la molécule: exemple de la molécule d'éthanol $CH_3$–CHD–OH

Pour pouvoir caractériser la configuration du carbone –CHD- par RMN du deutérium, il faut transformer l'alcool stéréospécifiquement en un composé diastéréoisomère. Ceci peut être fait par réaction de l'alcool avec l'acide camphanique (1) et addition ultérieure de dipivaloylméthane europium ou composé analogue:

(1) 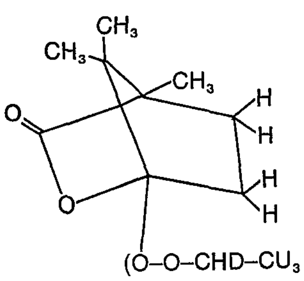

Deux signaux CHD (R) et CHD (S) apparaissent dans le spectre RMN et leur intensité relative donne accès à la pureté énantiomérique de l'éthanol considéré.

b) Cas où il y a un autre centre de dissymétrie dans la molécule: exemple de la molécule d'un amino-acide (proline)

La proline, comme la plupart des amino-acides, contient un carbone asymétrique (⊗)

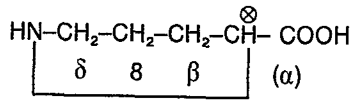

et les molécules naturelles monodeutériées existent sous forme de diastéréoisomères. Le spectre RMN du deutérium de la proline permet notamment d'identifier les diastéréoisomères ($\beta_R$) et ($\beta_S$) et ($\delta_R$) et ($\delta_S$):

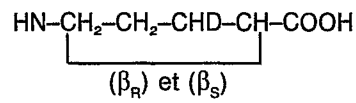

($\beta_R$) et ($\beta_S$)

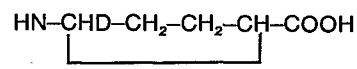

($\delta_R$) et ($\delta_S$)

La pureté énantiomérique des sites (β) et (δ) peut être déterminée par la mesure du rapport des intensités des signaux correspondants et être utilisée comme critère d'identification.

Exemples de calcul dans le cadre du procédé de reconnaissance de l'origine des molécules naturelles d'une espèce donnée

1er Exemple

Différenciation d'un alcool de maïs (A) et d'un alcool de betterave (B)

Un échantillon d'alcool industriel (96%) ou d'alcool extrait d'un breuvage alcoolisé obtenu par fermentation de maïs (A) ou par fermentation de mélasse de betterave (B) est introduit dans la cellule de mesure décrite ci-après et contenant de l'acétonitrile comme substance étalon. Les paramètres d'acquisition sont les suivants:

temps d'acquisition: 6,8 s
angle d'impulsion: 90°
gamme de balayage: 1200 Hz
découplage des protons par bruit: 3 watts
nombre d'impulsions: 2000
Multiplication exponentielle: 2 s

Les figures 2(A) et 2(B) montrent les spectres des deux alcools de maïs (A) et de betterave (B). Les résultats sont exprimés dans deux échelles différentes, correspondant aux hauteurs (h) et aux surfaces (s) des signaux.

| Signal | | (0) | (1) | (2) |
|---|---|---|---|---|
| A | h | 106,0 | 221,8 | 163,0 |
| | s | 26,8 | 44,7 | 32,4 |
| B | h | 133,5 | 206,0 | 184,0 |
| | s | 33,4 | 49,1 | 42,0 |

On en déduit les valeurs de D(1), D(2) et R

| | D(1) | D(2) | R |
|---|---|---|---|
| A(h) | 2,092 | 1,538 | 2,205 |
| B(h) | 1,543 | 1,378 | 2,680 |

La comparaison des valeurs D(1), D(2) et R permet d'identifier sans ambiguïté les deux alcools (A) et (B). On en déduit aussi que la teneur globale en deutérium de l'alcool de maïs (A) est 1,060 fois plus élevée que celle de l'alcool de betterave.

2ème Exemple

Identification d'un mélange d'alcools d'origines différentes dans un alcool de table commercial (whisky, vodka)

– un whisky «blended» est caractérisé par un mélange d'alcool d'orge (malt) et d'un autre alcool de grain (maïs par exemple). Soit $\bar{R}_{orge} = 2{,}480$ et $\bar{R}_{(maïs)} = 2{,}230$, valeurs qui correspondent respectivement à du whisky pur malt et du whisky pur grain de maïs.

On mesure la valeur R = 2,418 pour un whisky «blended» et on peut donc affirmer que ce whisky contient 25% d'alcool de maïs;

– une vodka du commerce est caractérisée par une valeur R = 2,627 et on sait, à la suite de mesures sur des vodkas d'origine contrôlée, qu'une vodka pur grain de blé ou pure pomme de terre est définie par la valeur $\bar{R}_{(blé)} = 2{,}470$ ou $\bar{R}_{(pomme\ de\ terre)} = 2{,}710$. La vodka commerciale contient donc 65% d'alcool de pomme de terre.

3ème Exemple

Identification d'une bière pur orge et d'une bière préparée avec un mélange d'orge et de grain cru (maïs par exemple)

En retenant pour l'alcool d'orge et de maïs les valeurs $\bar{R}_{(orge)} = 2{,}480$ et $\bar{R}_{(maïs)} = 2{,}230$, une bière caractérisée par un paramètre R = 2,405 sera réputée contenir 30% d'alcool de maïs. Une bière réputée pour orge devrait être caractérisée par une valeur comprise entre les limites R = 2,480 ± 0,02 (dans le cas de l'espèce d'orge considéré ici).

4ème Exemple

Différenciation des anétholes

La formule chimique de l'anéthole étant

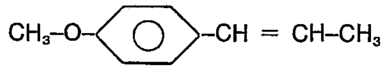

on a les six molécules mono deutériées différentes suivantes:

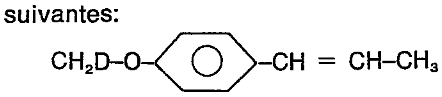 (1)

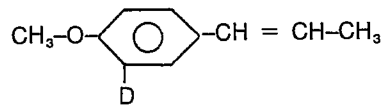 (2)

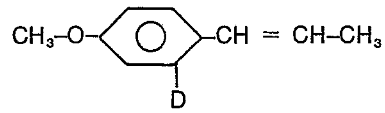 (3)

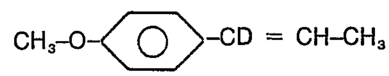 (4)

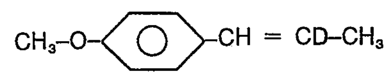 (5)

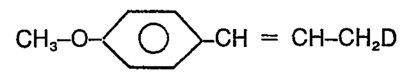 (6)

On calcule ainsi $D'_{(1)} = \frac{S(1)}{S(o)}$ $D'_{(2)} = \frac{S(2)}{S(o)}$, etc...

et de même T =

$$\frac{S(1) + S(2) + S(3) + S(4) + S(5) + S(6)}{S_o}$$

Le produit est introduit directement dans la cellule de mesure décrite ci-après et contenant de l'acétonitrile comme substance étalon. Les paramètres d'acquisition sont les mêmes que ceux qui ont été décrits dans l'Exemple 1.

Les figures 4(A) et 4(B) représentent les spectres des deux anétholes A (naturel) et B (synthétique).

| Signal | | (0) | (1) | (2) | (3) | (4) | (5) | (6) |
|---|---|---|---|---|---|---|---|---|
| A | h | 237,0 | 144,5 | 58,0 | 81,8 | 36,0 | 38,0 | 136,5 |
| | s | 40,0 | 30,0 | 24,0 | 30 | 13,5 | 11,2 | 27,8 |
| B | h | 209,0 | 152,2 | 52,3 | 73,0 | 53,2 | 34,0 | 175,7 |
| | s | 40,0 | 32,0 | 22,8 | 26,8 | 18,5 | 10,0 | 36,0 |

On en déduit les valeurs D(i) préalablement définies:

| | D(1) | D(2) | D(3) | D(4) | D(5) | D(6) | D(1)/D(6) |
|---|---|---|---|---|---|---|---|
| A(h) | 0,610 | 0,245 | 0,345 | 0,152 | 0,160 | 0,576 | 1,059 |
| B(h) | 0,728 | 0,250 | 0,349 | 0,255 | 0,163 | 0,841 | 0,866 |

La considération de la somme des surfaces des signaux d'un anéthole par rapport à ceux de la substance étalon permet de conclure à l'existence d'une teneur globale en deutérium 1,071 fois plus grande dans (B) que dans (A). Ces caractéristiques permettent d'identifier sans aucune ambiguïté les deux anétholes d'origine différente.

5ème Exemple

Identification de l'origine d'un amido-acide commercial ou naturel ou issu de la dégradation de protéines animales ou végétales. Cas de l'acide aspartique $HOCO-CH_2-CH(NH_2)COOH$.

L'acide aspartique est dissous dans de l'eau dépourvue de deutérium ($D/H \leq 1{,}5\ 10^{-6}$) et le pH de la solution est porté à 12 au moyen de petits grains de sodium. Les fractions molaires des différentes molécules deutériées présentes sont alors mesurées:

$HOCO-CHD-CH(NH_2)-COOH$ (R) $(\beta_R)$

$HOCO-CHD-CH(NH_2)-COOH$ (S) $(\beta_s)$

$HOCO-CH_2-CD(NH_2)-COOH$ $(\alpha)$

(les molécules contenant du deutérium sur les sites échangeables ne sont pas considérées ici).

Dans le cas d'une répartition statistique du deutérium, les fractions molaires fm de trois molécules $(\beta_R)$, $(\beta_S)$ et $(\alpha)$ sont égales à 0,143.

Un échantillon obtenu aux ETATS-UNIS est caractérisé par les valeurs

$fm(\beta_R) = 0{,}340$ $fm(\beta_S) = 0{,}245$ $fm(\alpha) = 0{,}415$

et un échantillon obtenu par fermentation en FRANCE.

$fm(\beta_R) = 0{,}330$ $fm(\beta_S) = 0{,}300$ $fm(\alpha) = 0{,}370$

La composition isotopique des sites $\beta$ par rapport au site $\alpha$ et la répartition des molécules chirales $(\beta_R)$ et $(\beta_S)$ diffèrent très significativement dans les deux échantillons qui peuvent être identifiés et caractérisés.

6ème Exemple

Détermination de l'année de production ou de récolte d'un échantillon par mesure des variations géo-climatiques du rapport isotopique R en fonction du lieu de production ou de récolte.

Une série d'alcools de betteraves, récoltées dans le Pas-de-Calais (F) entre 1972 et 1976, est caractérisée par la relation suivante:

$$\overline{R}_c = 4{,}68 + 0{,}00264\ H - 0{,}62\ T$$

($\overline{R}_c$ est la valeur réduite et centrée du paramètre $R_h$, H est la hauteur d'eau moyenne des précipitations atmosphériques du lieu de production et T la température moyenne du lieu de production pendant les mois de végétation de l'année considérée, H en mm et T en °C).

Un alcool de betteraves de millésime inconnu est caractérisé par une valeur $R_c = 1{,}250$. La consultation des tables de la Météorologie Nationale pour la région considérée permet de déduire que les valeurs de H et T de l'année 1975 donnent le meilleur accord de $R_c$ calculé avec $R_c$ expérimental.

Enregistrement des spectres RMN

a) Acquisition des spectres

La résolution doit être soigneusement réglée en résonance $^1H$, par utilisation du canal de verrouillage champ-fréquence. Une largeur de raie de l'ordre de 1 hertz peut alors être obtenue. Le nombre d'accumulations doit être suffisamment grand pour que le rapport signal/bruit soit compris entre 80 et 120; dans ces conditions, les mesures quantitatives relatives sont optimisées. 1000 à 2500 acquisitions sont suffisantes. Des valeurs plus faibles de rapport signal/bruit peuvent aussi être utilisées, mais la précision est légèrement diminuée. La longueur de l'impulsion correspond à un angle d'excitation de 90°.

Le temps d'acquisition doit assurer une bonne résolution digitale (0,07 à 0,015 Hz par point) sur une gamme restreinte. On peut utiliser des temps d'acquisition de 6,8 à 13,6 s pour des gammes de 600 à 1200 Hz.

Pour améliorer la précision statistique des mesures, il est recommandé d'accumuler 4000 à 6000 signaux d'induction libre et de les stocker sur disque magnétique en blocs de taille variable (1000 à 2000) selon l'accord de la sonde.

b) Manipulation du signal d'induction libre

Les mesures sont effectuées par séries de 3 à 6 spectres, qui sont stockées sur disque magnétique sous forme de signaux d'induction libre.

Chaque signal est ensuite transformé en spectre de fréquence, en utilisant une multiplication exponentielle variable. La raie ne doit pas être trop fine, pour éviter les anomalies de hauteur, ni trop large. Une constante de temps comprise entre 0 et 4 s est sélectionnée. Le spectre est ensuite digitalisé en fréquence, et intensité (hauteurs et intégrales), en utilisant un réglage de phase dans des conditions standard. Un tracé sur papier est effectué avec une dilatation de 1 à 12 Hz/cm et avec une vitesse de balayage précise.

Il est aussi possible de reproduire par photocopie les signaux sur papier de forte densité, de les découper et de les peser avec précision, en présence d'un carré de papier de surface constante servant de référence de densité de papier pour chaque signal. Dans le cas de courbes RMN larges, l'usage d'un planimètre est aussi recommandé.

Les signaux stockés par blocs sont multipliés en utilisant deux constantes de temps différentes (l'une comprise entre 0,5 et 1,51 et l'autre comprise entre 1,8 et 2,51) et une transformation de Fourier avec remplissage des mots de mémoire non utilisés par le signal à concurrence de 32K, est effectuée. Les spectres en fréquence sont

alors traités de deux façons différentes, à partir de la liste des intensités donnée par le calculateur sur le spectre d'absorption avec réglage de phase standard et sur le spectre puissance, et à partir du spectre enregistré sur papier qui permet aussi de corriger la ligne de base des fréquences et de l'intégrale. Un traitement statistique sur 18 à 36 valeurs d'intensités est effectué pour obtenir la moyenne et l'écart-type de la population de mesures, supposée normale. Pour un degré de confiance de 99%, la valeur moyenne du paramètre $R_h$ est encadrée dans les limites $\overline{R}_h \pm 0{,}005$.

Lorsqu'il s'agit de comparer plusieurs échantillons d'une série d'alcool, un traitement d'analyse factorielle améliore encore la précision des mesures. Les données sont arrangées sous forme d'une matrice à m lignes et n colonnes; les colonnes n représentent les différents échantillons, pour chacun desquels 18 à 36 mesures du rapport $R_h$ sont disponibles (les rapports $R_h$ représentent donc les lignes de la matrice d'observation). La matrice décomposée en facteurs indépendants est ensuite reconstruite avec le premier facteur qui correspond à la plus grande valeur propre, largement dominante (supérieure à 95%). Les nouvelles valeurs de $R_h$ ainsi obtenues sont dégagées des erreurs aléatoires liées aux mesures et permettent une comparaison plus sensible des différents échantillons de la série.

Description d'une cellule de mesure

La cellule de mesure conforme à la présente invention, représentée à la figure 1, est constituée de deux tubes coaxiaux: le tube coaxial 10 extérieur en verre pyrex calibré de diamètre extérieur de 15 mm, et le tube coaxial 11 intérieur en verre pyrex calibré, de diamètre extérieur de 5 mm. Ce dernier est représenté scellé après l'introduction d'étalon. Les deux tubes sont séparés et maintenus solidaires grâce à deux joints en Téflon: le bloc supérieur 15 et le bloc inférieur 16. Le bloc de Téflon cylindrique supérieur de diamètre extérieur de 13,45 mm, est percé en son centre par un trou de diamètre intérieur égal à 5 mm sur toute sa longueur (environ 3 cm), afin de pouvoir y loger le tube 11. Ce bloc 15 est également percé de deux orifices 12 et 13, de diamètre intérieur de 2 mm et filetés sur 1 cm. L'orifice 12 est destiné à introduire dans la partie inférieure 17 du tube 10, l'échantillon à analyser à l'aide par exemple d'une seringue et d'une aiguille. L'air contenu dans la partie inférieure 17 s'échappe par l'orifice 13. Le tube intérieur 11 repose sur le bloc 16 dans lequel on a percé un trou borgne de diamètre intérieur de 5 mm, sur une longueur de 2 cm, afin d'y loger la partie inférieure du tube 11.

L'orifice 14 percé dans le bloc 16 permet à l'air de s'échapper du fond du tube lors de la descente du bloc. Le trou est aussi fileté de façon à pouvoir extraire le bloc du tube au moyen de la tige filetée. Les blocs 15 et 16 sont usinés de façon à produire des saignées cylindriques qui facilitent le glissement Téflon-Pyrex.

Cette cellule peut convenir parfaitement pour des quantités de liquide allant de 1 à 15 ml.

**Revendications**

1. Procédé de détection et de différenciation qualitative et quantitative des molécules naturellement deutériées, et notamment procédé de détection de la chaptalisation des vins, caractérisé en ce que:

a) l'on prépare un étalon en mélangeant un produit hydrocarboné du commerce avec son analogue deutérié artificiellement à 95–99%, de manière à ce que le taux de deutériation global du mélange soit compris entre 0,01 et 0,2%,

b) l'on introduit cet étalon dans une cellule de mesure de l'appareil d'analyse par résonance magnétique nucléaire (RMN), ladite cellule étant constituée de telle façon qu'elle puisse recevoir l'étalon de manière à ce qu'il n'y ait aucun mélange entre l'étalon et le produit à analyser,

c) l'on ajoute dans la cellule le produit à analyser, et

d) l'on introduit la cellule dans l'appareil RMN(D) et l'on enregistre le spectre du deutérium que l'on compare au spectre RMN(D) des produits de provenance géographique et de sources chimiques et/ou biochimiques connues, réalisé auparavant avec le même étalon.

2. Procédé selon la Revendication 1, caractérisé en ce que dans le cas des alcools, l'étalon est constitué par une référence intramoléculaire, laquelle est chimiquement combinée à l'alcool et le produit résultant est introduit dans la cellule et soumis à l'étape b) de la Revendication 1.

3. Procédé selon la Revendication 1, caractérisé en ce que pour déterminer la pureté énantiomérique des alcools, on combine chimiquement l'alcool à un composé optiquement actif et on introduit ce composé dans la cellule selon b) de la Revendication 1.

4. Procédé selon la Revendication 1, caractérisé en ce qu'on choisit l'étalon parmi les produits suivant le tétraméthylsilane, l'hexaméthyldisilane, l'hexaméthyldisiloxane, l'hexaméthyldisilazane, le diméthyl-4,4-silapentane-4-sulfonate de sodium, le diméthyl-4,4-silapentane-4-carboxylate de sodium, l'octaméthylcyclotétrasiloxane, le tétrakis-(triméthylsilyl)méthane, l'acétonitrile, l'acétone, le benzène, le diméthylsulfoxyde et le chloroforme.

5. Procédé selon l'une quelconque des Revendications 1 et 4, caractérisé en ce qu'on ajoute à l'étalon et/ou à l'échantillon à analyser, des quantités faibles de chélates du chrome et/ou du fer et/ou du cobalt et/ou du nickel et/ou d'europium et/ou du praséodyme et/ou d'ytterbium et/ou de dysprosium et/ou du holmium et/ou d'erbium.

6. Procédé selon la Revendication 5, caractérisé en ce que lesdits chélates sont préparés à partir d'une dione de formule générale I ci-après:

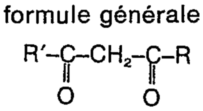

(I)

dans laquelle:

R représente: $CH_3$, $tC_4H_9$, $nC_3F_7$, $C_2F_5$, $CF_3$, $tC_4D_9$, et

R′ représente: $CH_3$, $tC_4H_9$, $CF_3$, $nC_3F_7$, $C_2F_5$, $nC_3H_7$.

7. Procédé selon l'une quelconque des Revendications 1 à 6, caractérisé en ce que dans le cas où l'analyse porte sur des vins, on procède auparavant à une distillation après la neutralisation à pH 7,5 à l'aide d'un alcali.

8. Procédé selon l'une quelconque des Revendications 1 à 7, caractérisé en ce qu'on déshydrate tout d'abord l'échantillon sur du $CaH_2$, puis on distille par cryopiégeage à l'azote liquide.

9. Procédé selon l'une quelconque des Revendications 1 à 8, caractérisé en ce qu'on ajoute à l'échantillon 0,01 et 1% vol/vol d'un acide pris dans le groupe qui comprend l'acide chlorhydrique, l'acide nitrique, l'acide sulfurique, l'acide phosphorique, l'acide trichloracétique et l'acide perchlorique.

10. Procédé selon l'une quelconque des Revendications 2, 3, 5 à 9, caractérisé en ce que la référence intramoléculaire est constituée à partir d'anhydride acétique, et/ou d'acide acétique, et/ou de chlorure d'acétyle, et/ou de bromure d'acétyle, le produit résultant est alors de l'acétate d'éthyle.

11. Procédé selon l'une quelconque des Revendications 1 à 10, caractérisé en ce que le composé optiquement actif est constitué d'acide camphanique et/ou d'acide malique et/ou d'acide aspartique et/ou de proline et/ou de phénylalanine, auxquels on ajoute des quantités faibles de chelates.

12. Dispositif pour la mise en œuvre du procédé selon l'une quelconque des Revendications 1 à 11, caractérisé en ce qu'il est constitué d'une cellule de mesure par RMN constituée de deux tubes coaxiaux (10, 11) isolés l'un de l'autre, et dont le rapport

$$\frac{\text{diamètre tube extérieur}}{\text{diamètre tube intérieur}}$$

est compris entre 2,5:1 à 3,5:1, le tube intérieur (11) maintenu solidaire du tube extérieur (10) par des joints de solidarité (15, 16) étant destiné à recevoir l'étalon et pouvant être scellé après l'introduction de celui-ci, tandis que le tube extérieur (10) est destiné à recevoir la substance à analyser introduite dans la partie inférieure (17) dudit tube extérieur, à travers un orifice (12) percé dans le joint de solidarité (15) qui maintient les deux tubes.

13. Dispositif selon la Revendication 12, caractérisé en ce que ladite cellule est dotée d'un guide gradué et étalonné en fonction du spectromètre utilisé, lequel guide permet de régler la hauteur de la turbine sur le tube extérieur selon la quantité de la substance contenue dans la partie inférieure de ce tube extérieur.

14. Dispositif selon l'une quelconque des Revendications 12 et 15, caractérisé en ce que les deux tubes coaxiaux (10, 11) sont en verre pyrex calibré.

15. Dispositif selon l'une quelconque des Revendications 12 à 14, caractérisé en ce que les joints de solidarité (15, 16) sont au nombre de deux, l'un inférieur (16) maintenant le tube intérieur sur sa base, et l'autre supérieur (15) maintenant le tube intérieur en son milieu.

16. Dispositif selon la Revendication 15, caractérisé en ce que les deux joints de solidarité sont constitués de deux blocs de Téflon cylindriques.

17. Dispositif selon l'une quelconque des Revendications 15 et 16, caractérisé en ce que les deux blocs sont percés: le bloc supérieur de deux orifices, l'un (12) pour l'introduction de la substance à analyser, et l'autre (13) pour l'échappement de l'air, tandis que le bloc inférieur est percé d'un orifice (14) pour fixer le tube intérieur contenant la substance étalon.

18. Dispositif selon l'une quelconque des Revendications 12 à 17, caractérisé en ce que les deux blocs cylindriques sont pourvus de saignées, afin de permettre un glissement aisé dans le tube extérieur (10).

19. Dispositif selon l'une quelconque des Revendications 15 à 18, caractérisé en ce que deux tiges filetées sont adaptables sur le bloc supérieur de solidarité, de manière à régler exactement l'affleurement de la substance à analyser contenue dans la partie inférieure du tube extérieur, à la surface inférieure dudit bloc de solidarité.

20. Dispositif selon la Revendication 12, caractérisé en ce que le diamètre des tubes coaxiaux est compris entre 10 et 50 mm pour le tube extérieur (10), et entre 4 et 12 mm pour le tube intérieur (11).

**Claims**

1. A process for the detection and quantitative and qualitative differentiation of naturally deuterated molecules, and more especially a process for detecting the chaptalization of wines, characterized in that:

a) a standard is prepared by mixing a commercially available hydrocarbonic product with its analogue deuterated artificially to 95–99%, so that the overall deuteration rate of the mixture is between 0.01 and 0.2%,

b) this standard is introduced into a measuring cell of the nuclear magnetic resonance analysis apparatus (RMN), said cell being formed so that it may receive the standard so that there is no mixing between the standard and the products to be analyzed,

c) the product to be analyzed is added into the cells and

d) the cell is introduced into the RMN(D) apparatus and the spectrum of the deuterium is recorded which is compared with the RMN(D) spectrum of the products of known geographical origin and known chemical and/or biochemical sources, made beforehand with the same standard.

2. The process according to claim 1, characterized in that the case of alcohols, the standard is constituted by an intramolecular reference which is chemically combined with the alcohol and the resulting product is introduced into the cell and subjected to step b) of claim 1.

3. The process according to claim 1, characterized in that the enantiomeric purity of alcohols is determined by combining them chemically with

an optically active compound and the resulting product is introduced into the cells and subjected to step b) of claim 1.

4. The process according to claim 1, characterized in that the standard is chosen from the products of the following group: tetramethylsilane, hexamethyldisilane, hexamethyldisiloxane, hexamethyldisilazane, 4,4-dimethyl-4-silapentane-sodium sulfonate, 4,4-dimethyl-4-silapentane sodium carboxylate, octamethylcyclotetrasiloxane, tetrakis-(trimethylsilyl)methane, acetonitrile, acetone, benzene, dimethylsulfoxyde and chloroform.

5. The process according to any one of claims 1 and 4, characterized in that there is added to the standard and/or to the sample to be analyzed, small amounts of chromium and/or iron and/or cobalt and/or nickel and/or europium and/or praseodyme and/or ytterbium and/or dysprosium and/or holmium and/or erbium chelates.

6. The process according to claim 5, characterized in that said chelates are prepared from a dione of the general formula I below:

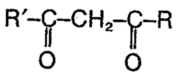
(I)

in which:

R represents: $CH_3$, $tC_4H_9$, $nC_3F_7$, $C_2F_5$, $CF_3$, $tC_4D_9$, and

R′ represents: $CH_3$, $tC_4H_9$, $nC_3F_7$, $C_2F_5$, $nC_3H_7$, $CF_3$.

7. The process according to any one of claims 1 to 6, characterized in that, in the case where the analysis relates to wines, distillation is carried out beforehand after neutralization to pH 7.5 with an alkali.

8. The process according to any one of claims 1 to 7, characterized in that the sample is first of all dehydrated on $CaH_2$, then distilled by liquid nitrogen cryo-trapping.

9. The process according to claims 1 to 8, characterized in that there is added to the sample between 0.01 and 1% vol/vol of an acid chosen from the group comprising hydrochloric acid, nitric acid, sulfuric acid, phosphoric acid, trichloracetic acid and perchloric acid.

10. The process according to any one of claims 2, 3, 5 to 9, characterized in that the intramolecular reference is formed from acetic anhydride and/or acetic acid and/or acetyl chloride and/or acetyl bromide, the resulting product is then ethyl acetate.

11. The process according to any one of claims 1 to 10, characterized in that the optically active compound is formed from camphanic acid and/or malic acid and/or aspartic acid and/or proline and/or phenylalanine, to which small amounts of chelates are added.

12. A device for implementing the process according to any one of claims 1 to 11, characterized in that it is formed of an RMN measuring cell formed from two coaxial tubes (10, 11) insulated from each other and whose

$$\frac{\text{external tube diameter}}{\text{internal tube diameter}} \text{ ratio}$$

is between 2.5:1 and 3.5:1, the inner tube (11) being firmly secured to the outer tube (10) by securing seals (15, 16) being intended to receive the standard and being able to be sealed after introduction thereof, whereas the outer tube (10) is intended to receive the substance to be analyzed, introduced into the lower part (17) of said external tube, through an orifice (12) pierced in the securing seal (15) which holds the two tubes in position.

13. The device according to claim 12, characterized in that said cell is provided with a guide graduated and calibrated as a function of the spectrometer used, which guide allows the height of the turbine on the outer tube to be adjusted depending on the amount of the substance contained in the lower part of this outer tube.

14. The device according to any one of claims 12 and 13, characterized in that the two coaxial tubes (10, 11) are made from gauged Pyrex glass.

15. The device according to any one of claims 12 to 14, characterized in that the securing seals (15, 16) are two in number, the lower one (16) holding the inner tube at its base and the upper one (15) holding the inner tube at its middle.

16. The device according to claim 15, characterized in that the two securing seals are formed from two cylindrical Teflon blocks.

17. The device according to any one of claims 15 and 16, characterized in that the two blocks are pierced: the upper block with two orifices, one (12) for introducing the substances to be analyzed and the other (13) for letting the air escape, whereas the lower block is pierced with one orifice (14) for fixing the inner tube containing the standard substance.

18. The device according to any one of claims 12 to 17, characterized in that the two cylindrical blocks are provided with grooves, so as to allow easy sliding in the other tube (10).

19. The device according to any one of claims 15 to 18, characterized in that two threaded rods are adaptable to the upper securing block, so as to exactly adjust the levelling of the substance to be analyzed contained in the lower part of the outer tube with the lower surface of said securing block.

20. The device according to claim 12, characterized in that the diameter of the coaxial tubes is between 10 and 50 mm for the outer tube (10) and between 4 and 12 mm for the inner tube (11).

**Patentansprüche**

1. Verfahren zum Nachweisen und zur qualitativen und quantitativen Differenzierung von natürlich deuterierten Molekülen, insbesondere Verfahren zum Nachweisen der Aufzuckerung von Weinen, dadurch gekennzeichnet, dass:

a) man einen Standard herstellt, indem man ein handelsübliches Kohlenwasserstoffprodukt mit seinem zu 95–99% künstlich deuteriertem Analogon derart mischt, dass der Gesamtdeuterisationsgrad der Mischung zwischen 0,01 und 0,2% liegt,

b) man diesen Standard in eine Messzelle eines Analyseapparats mit kernmagnetischer Resonanz

(KMR) einbringt, welche Zelle derart ausgebildet ist, dass sie den Standard so aufnehmen kann, dass keine Vermischung zwischen dem Standard und dem zu analysierenden Produkt stattfindet,

c) man das zu analysierende Produkt in die Zelle gibt, und

d) man die Zelle in den KMR(D)-Apparat einbringt und das Spektrum des Deuteriums aufzeichnet, das man mit dem KMR(D)-Spektrum von Produkten bekannter geografischer Herkunft und chemischer und/oder biochemischer Quellen, welches zuvor mit demselben Standard erstellt worden ist, vergleicht.

2. Verfahren nach Anspruch 1, dadurch gekennzeichnet, dass im Fall von Alkoholen der Standard durch eine intramolekulare Referenz gebildet wird, welche an den Alkohol chemisch gebunden wird, und das resultierende Produkt in die Zelle eingebracht und der Stufe b) des Anspruchs 1 unterworfen wird.

3. Verfahren nach Anspruch 1, dadurch gekennzeichnet, dass man zur Bestimmung der enantiomeren Reinheit der Alkohole den Alkohol an eine optisch aktive Verbindung chemisch bindet und diese Verbindung gemäss b) des Anspruchs 1 in die Zelle einbringt.

4. Verfahren nach Anspruch 1, dadurch gekennzeichnet, dass man den Standard unter den folgenden Produkten auswählt: Tetramethylsilan, Hexamethyldisilan, Hexamethyldisiloxan, Hexamethyldisilazan, 4,4-Dimethyl-4-silapentan-natriumsulfonat, 4,4-Dimethyl-4-silapentan-natriumcarboxylat, Octamethylcyclotetrasiloxan, Tetrakis-(trimethylsilyl)-methan, Acetonitril, Aceton, Benzol, Dimethylsulfoxid und Chloroform.

5. Verfahren nach einem der Ansprüche 1 und 4, dadurch gekennzeichnet, dass man geringe Mengen von Chrom- und/oder Eisen- und/oder Kobalt- und/oder Nickel- und/oder Europium- und/oder Praseodym- und/oder Ytterbium- und/oder Dysprosium- und/oder Holmium- und/oder Erbiumchelaten zum Standard und/oder zu der zu analysierenden Probe zugibt.

6. Verfahren nach Anspruch 5, dadurch gekennzeichnet, dass die Chelate ausgehend von einem Dion der nachstehenden Formel I:

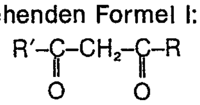

(I)

hergestellt werden, worin

R bedeutet: $CH_3$, $tC_4H_9$, $nC_3F_7$, $C_2F_5$, $CF_3$, $tC_4D_9$, und

R′ bedeutet: $CH_3$, $tC_4H_9$, $CF_3$, $nC_3F_7$, $C_2F_5$, $nC_3H_7$.

7. Verfahren nach einem der Ansprüche 1 bis 6, dadurch gekennzeichnet, dass man im Falle einer Weinanalyse zuvor eine Destillation nach Neutralisation auf pH 7,5 mit Hilfe eines Alkalis vornimmt.

8. Verfahren nach einem der Ansprüche 1 bis 7, dadurch gekennzeichnet, dass man zuerst die Probe über $CaH_2$ dehydratisiert und dann mittels Kühlfalle mit flüssigem Stickstoff destilliert.

9. Verfahren nach einem der Ansprüche 1 bis 8, dadurch gekennzeichnet, dass man zur Probe zwischen 0,01 und 1% V./V. einer Säure aus der Gruppe umfassend Salzsäure, Salpetersäure, Schwefelsäure, Phosphorsäure, Trichloressigsäure und Perchlorsäure, zugibt.

10. Verfahren nach einem der Ansprüche 2, 3, 5 bis 9, dadurch gekennzeichnet, dass die intramolekulare Referenz ausgehend von Essigsäureanhydrid und/oder Essigsäure und/oder Acetylchlorid und/oder Acetylbromid gebildet wird, wobei das resultierende Produkt dann Äthylacetat ist.

11. Verfahren nach einem der Ansprüche 1 bis 10, dadurch gekennzeichnet, dass die optisch aktive Verbindung aus Camphansäure und/oder Apfelsäure und/oder Asparaginsäure und/oder Prolinsäure und/oder Phenylalaninsäure, zu welchen man geringe Mengen von Chelaten zugibt, gebildet wird.

12. Vorrichtung zur Durchführung des Verfahrens nach einem der Ansprüche 1 bis 11, dadurch gekennzeichnet, dass sie durch einer KMR-Messzelle mit zwei koaxialen, voneinander getrennten Rohren gebildet ist, deren Verhältnis

$$\frac{\text{Durchmesser Aussenrohr}}{\text{Durchmesser Innenrohr}}$$

zwischen 2,5:1 und 3,5:1 beträgt, wobei das Innenrohr (11), das mittels Abstandhaltern (15, 16) am Aussenrohr (10) festgehalten ist, zur Aufnahme des Standards dient und nach Einführung desselben verschliessbar ist, während das Aussenrohr (10) zur Aufnahme der zu analysierenden Substanz dient, die in den unteren Teil (17) des Aussenrohres durch eine in dem die beiden Rohre haltenden Abstandhalter (15) gebohrte Öffnung (12) eingebracht wird.

13. Vorrichtung nach Anspruch 12, dadurch gekennzeichnet, dass die Zelle mit einer kalibrierten und je nach verwendetem Spektrometer geeichten Führung ausgestattet ist, welche Führung die Regulierung der Höhe der Turbine am Aussenrohr entsprechend der Menge der im unteren Teil dieses Aussenrohrs enthaltenen Substanz gestattet.

14. Vorrichtung nach einem der Ansprüche 12 und 15, dadurch gekennzeichnet, dass die beiden koaxialen Rohre (10, 11) aus kalibriertem Pyrexglas sind.

15. Vorrichtung nach einem der Ansprüche 12 bis 14, dadurch gekennzeichnet, dass es zwei Abstandhalter (15, 16) gibt, einen unteren (16), der das Innenrohr an der Basis hält, und einen oberen (15), der das Innenrohr in dessen Mitte hält.

16. Vorrichtung nach Anspruch 15, dadurch gekennzeichnet, dass die beiden Abstandhalter durch zwei zylindrische Teflonblöcke gebildet sind.

17. Vorrichtung nach einem der Ansprüche 15 und 16, dadurch gekennzeichnet, dass die beiden Blöcke durchbohrt sind: der obere Block von zwei Löchern, einem (12) zum Einbringen der zu analysierenden Substanz und einem anderen (13) zum Auslassen von Luft, während der untere Block von einem Loch (14) zum Fixieren des die Standardsubstanz enthaltenden Innenrohrs durchbohrt ist.

18. Vorrichtung nach einem der Ansprüche 12 bis 17, dadurch gekennzeichnet, dass die beiden zylindrischen Blöcke zur Erleichterung des Gleitens im Aussenrohr (10) mit Schlitzen versehen sind.

19. Vorrichtung nach einem der Ansprüche 15 bis 18, dadurch gekennzeichnet, dass zwei Gewindestangen an den oberen Halteblock anpassbar sind, um die Nivellierung der im unteren Teil des Aussenrohrs enthaltenen zu analysierenden Substanz an der Unterseite des Halteblocks exakt zu regulieren.

20. Vorrichtung nach Anspruch 12, dadurch gekennzeichnet, dass der Durchmesser der koaxialen Rohre zwischen 10 und 50 mm beim Aussenrohr (10) und zwischen 4 und 12 mm beim Innenrohr (11) liegt.

FIG. 4

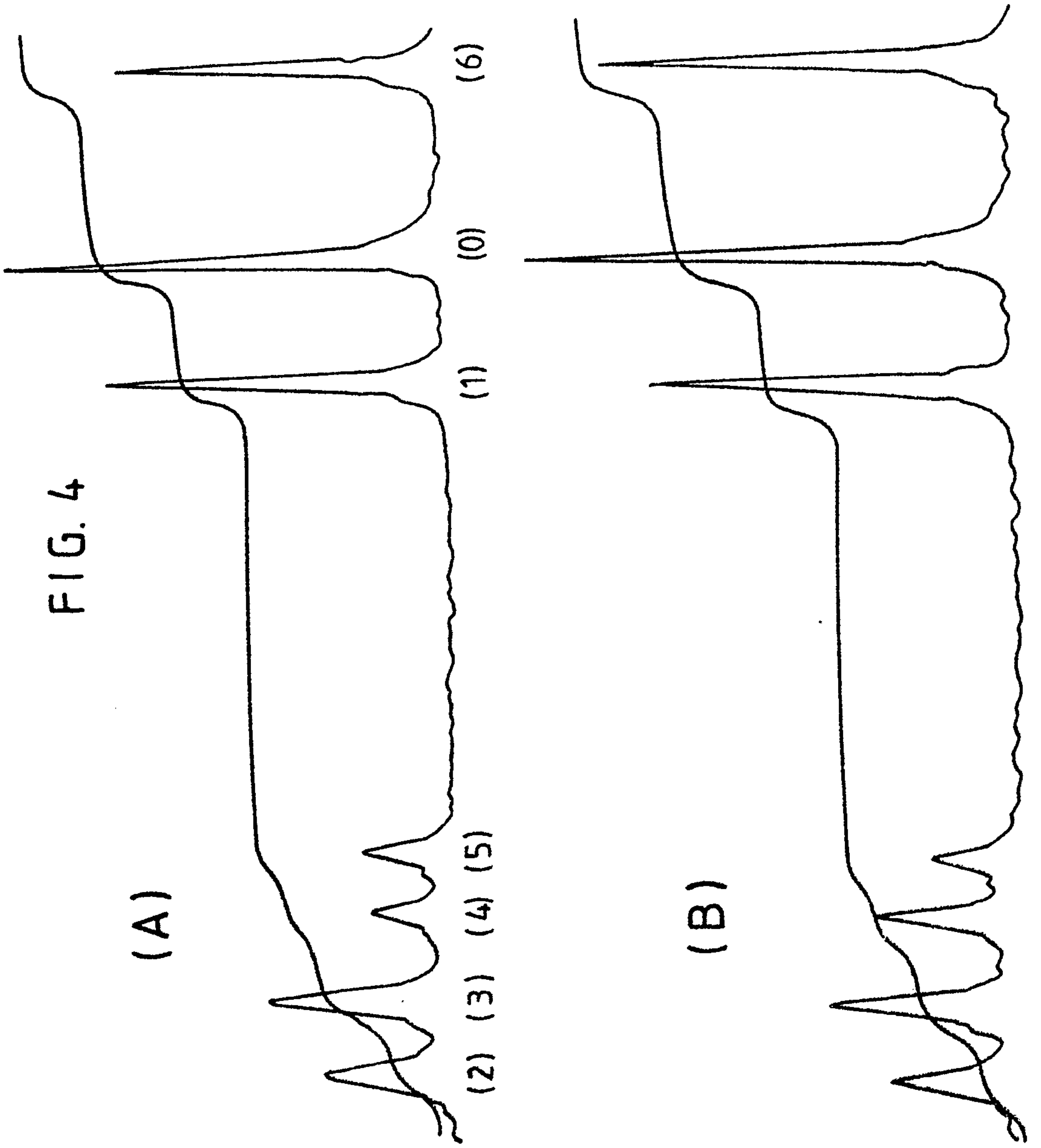


FIG.1

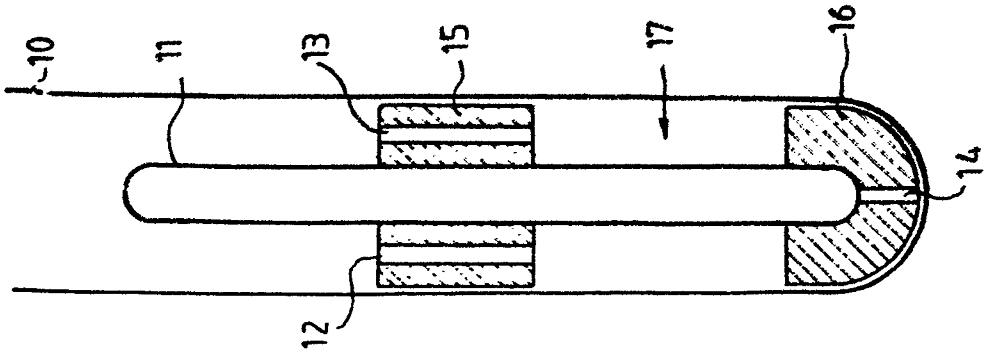

Fig. 2

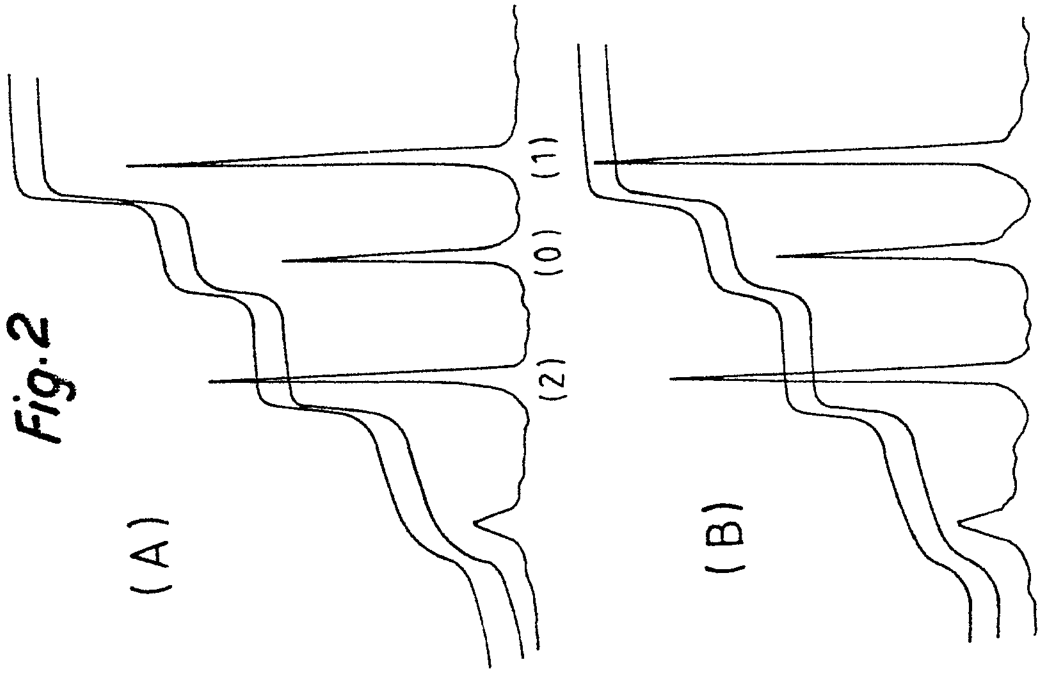


Fig. 3